Europäisches Patentamt

European Patent Office

Office européen des brevets

(11) Veröffentlichungsnummer: **0 358 939**
**A2**

(12) ## EUROPÄISCHE PATENTANMELDUNG

(21) Anmeldenummer: 89114627.6

(22) Anmeldetag: 08.08.89

(51) Int. Cl.5 **C12N 9/00**

(30) Priorität: 12.08.88 DE 3827392

(43) Veröffentlichungstag der Anmeldung:
21.03.90 Patentblatt 90/12

(84) Benannte Vertragsstaaten:
BE CH DE FR GB IT LI NL

(71) Anmelder: HOECHST AKTIENGESELLSCHAFT
Postfach 80 03 20
D-6230 Frankfurt am Main 80(DE)

(72) Erfinder: Then, Johann, Dr.
Bechtenwaldstrasse 70
D-6230 Frankfurt am Main 80(DE)
Erfinder: Giani, Carlo, Dr.
Hedderichstrasse 69
D-6000 Frankfurt am Main 70(DE)
Erfinder: Wöhner, Gerhard, Dr.
Mühlenkampstrasse 77
D-3050 Wunstorf(DE)
Erfinder: Voelskow, Hartmut, Dr.
Akazienstrasse 22
D-6234 Hattersheim am Main(DE)
Erfinder: Fricke, Ulrich
Am Flachsland 56
D-6233 Kelkheim (Taunus)(DE)

(54) **Pilz-lysierendes Enzymprodukt aus Streptomyceten, dessen Herstellung und Verwendung.**

(57) Durch Kultivierung eines Streptomyceten auf einem Chitinhaltigen Nährboden kann ein Pilz-lysierendes Enzymprodukt gewonnen werden, das zur Konservierung mikrobiell verderblicher Produkte eingesetzt werden kann.

EP 0 358 939 A2

## Pilz-lysierendes Enzymprodukt aus Streptomyceten, dessen Herstellung und Verwendung

Die mikrobielle Produktion von Metaboliten mit deren Hilfe das Wachstum von Pilzen unterdrückt werden kann wurde verschiedentlich beschrieben. In dem US-patent 4 534 965 wird ein Mittel offenbart, mit dessen Hilfe phytopathogene Pilze abgetötet werden können. Dieses Mittel wird durch Kultivierung des Streptomyces-Stammes ATCC 39434 auf einem Chitin-haltigen Substrat gewonnen. Es wird angenommen, daß das Mittel in ähnlicher Weise wie Cycloheximid auf die Pilze wirkt.

In der Europäischen Patentanmeldung 0 171 381 werden Mikroorganismen beschrieben, mit deren Hilfe das Wachstum von Nematoden unterdrückt werden kann. Es werden Pseudomonaden mit fremden Genen, die für Chitinase und Glykosidase kodieren, transformiert, um durch höhere Enzymausschüttung eine stärkere wachstumsunterdrückende Wirkung auf die Nematoden ausüben zu können.

Ebenfalls ein mit einem Chitinase-Gen transformiertes Bakterium wird in der Europäischen Patentanmeldung 0 157 351 erwähnt.

Es wurde nun überraschend ein neuartiges Pilz-lysierendes Enzymprodukt in der Kulturbrühe eines Streptomyceten gefunden.

Die Erfindung betrifft somit:

1. Ein Pilz-lysierendes Enzymprodukt erhältlich durch Kultivierung von Streptomyces spec. DSM 4666

2. Ein Verfahren zur Herstellung des unter 1. charakterisierten Pilz-lysierenden Enzymprodukts, das dadurch gekennzeichnet ist daß Streptomyces spec. DSM 4666 auf einem Chitin-haltigen bzw. Oligomere von $\beta$-1,4-N-Acetyl-glucosamin enthaltenden Substrat kultiviert wird.

3. Die Verwendung des unter 1. charaktierisierten Pilzlysierenden Enzymprodukts zur Konservierung von mikrobiell verderblichen Produkten.

Im folgenden wird die Erfindung detailliert beschrieben, insbesondere in ihrem bevorzugten Ausführungsformen. Ferner wird die Erfindung in den Patentansprüchen definiert.

Das Pilz-lysierende Enzymprodukt kann durch Kultivierung von Streptomyces spec. DSM 4666 hergestellt werden, der bei der Deutschen Sammlung von Mikroorganismen mit der genannten Nummer unter den Bedingungen des Budapester Vertrages hinterlegt wurde.

Der erfindungsgemäß verwendete Streptomyces Stamm kann wie folgt charakterisiert werden:

| Sporenfarbe | grau |
| Sporenkette | gerade bis gewellt |
| Sporenoberfläche | glatt |
| Melanin | - |
| Pigmentbildung | - |
| Luft Myzelfarbe | weiß |

Anstelle des genannten Stamms können auch jeweils deren Mutanten und Varianten eingesetzt werden, sofern sie das Pilz-lysierendes Enzymprodukt synthetisieren. Solche Mutanten können in an sich bekannter Weise durch physikalische Mittel, beispielsweise Bestrahlung, wie mit Ultraviolett- oder Röntgenstrahlen oder chemische Mutagene, wie beispielsweise Ethylmethansulfonat (EMS), N-Methyl-N′-nitro-N-nitroso-guanidin (MNNG) oder 2-Hydroxy-4-methoxy-benzophenon (MOB) erzeugt werden.

Als bevorzugte Kohlenstoffquelle für die aerobe Fermentation eignet sich Chitin, beispielsweise aus Insekten, Arthropoden oder Pilzzellwänden. Es können auch Oligomere des N-Acetyl-glucosamins verwendet werden. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali-oder Erdalkalimetalle, Eisen, Zink und Mangan als zusätzliche anorganische Salze enthalten.

Die Bildung des erfindungsgemäßen Enzymprodukts verläuft besonders gut in einer Nährlösung, die 0,1 bis 50 g/l, bevorzugt 1 bis 10 g/l, Krabbenchitin enthält.

Die Fermentation erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 40°C, vorzugsweise bei etwa 25 bis 30°C, insbesondere bei 28 bis 30°C und in einem pH-Bereich von 5 bis 9, bevorzugt 6,5 bis 8, erfolgen.

Man kultiviert den Mikroorganismus unter den genannten Bedingungen bis die stationäre Phase erreicht ist, etwa 60 bis 120 Stunden bevorzugt 60 bis 75 Stunden.

Vorteilhaft kultiviert man in mehreren Stufen, d.h. man stellt zunächst eine oder mehrere Vorkulturen in einem flüssigen Nährmedium her, die dann in das eigentlich, Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielweise Hefe-Malz-Agar, wachsen läßt.

Der Fermentationsverlauf kann anhand des pH-Wertes der Kultur oder des Mycelvolumens, durch Ausprüfen der biologischen Aktivität überwacht werden.

Die Isolierung des Pilz-lysierenden Enzymprodukts kann erfolgen durch Ionenaustauscherchromatographie an stark basischen Anionenaustauschern wie beispielweise Amberlite-Harzen.

Das erfindungsgemäße Enzymprodukt ist wie folgt zu charakterisieren:
- Temperaturstabilität bis zu 70°C,
- Temperaturoptimum 40 bis 60°C, insbesondere 45 bis 55°C,
- pH-Stabilität von pH 4,0 bis 10,0, insbesondere von pH 5 bis 7,
- pH-Optimum im Bereich von 4,5 bis 6, insbesondere 5 bis 5,5.

Das Enzymprodukt kann zur Konservierung verderblicher Produkten eingesetzt werden, die durch Pilzbefall geschädigt werden können. Dazu gehören Saatgut, Lebensmittel und pharmazeutische Produkte.

In den folgenden Beispielen wird die Erfindung weitergehend erläutert. Prozentangaben beziehen sich, sofern nicht anders angegeben, auf das Gewicht.

**Beispiel 1**

Kultivierung von Streptomyces spec. DSM 4666

Streptomyces spec. DSM 4666 wurde auf Schrägagar-Röhrchen, die

| Glucose | 10 g/l | |
|---|---|---|
| Caseinpepton | 4 g/l | |
| Fleischextrakt | 40 g/l | |
| Hefeextrakt | 0,5 g/l | |
| Leberextrakt | 0,5 g/l | |
| NaCl | 2,5 g/l | |
| Agar | 18 g/l | pH 7,2 |

enthielten, 10 Tage bei 30°C kultiviert. Die Sporen wurden mit 3 ml 0,9 % NaCl-Lösung abgeschwemmt. Die gesamte Sporensuspension wurde als Impfmaterial für Schüttelkolben (300 ml Inhalt, 100 ml Füllung, obige Nährlösung ohne Agar) verwendet. Die Kultur wurde bei 30°C und 180 Umdrehungen pro Minute (UPM) geschüttelt. Nach 48 h wurde die Hauptkultur mit 3 ml beimpft. Die Hauptkultur hatte folgende Zusammensetzung:

| Krabbenchitin | 10 g/l |
|---|---|
| $KH_2PO_4$ | 0,3 g/l |
| $MgSO_4 \cdot 7\,H_2O$ | 0,5 g/l |
| $K_2HPO_4$ | 0,7 g/l |
| Spurenelementlösung | 5 ml |
| pH 7,3 | |

Spurenelementlösung:

3 g/l $CaCl_2 \cdot 2 H_2O$
1 g/l Fe-III-citrat
0.2 g/l $MnSO_4$
0,1 g/l $ZnCl_2$
0,025 g/l $CuSO_4 \cdot 5 H_2O$
0,02 g/l $Na_2B_4O_9 \cdot 10 H_2O$
0,004 g/l $CoCl_2$
0,01 g/l $Na_2MoO_4 \cdot 2 H_2O$

Es wurde unter den gleichen Bedingungen wie oben kultiviert. Nach 3 Tagen wurden die Zellen abzentrifugiert und im Überstand die Enzymaktivität bestimmt. Sie betrug 123 U/l

**Beispiel 2**

Aktivitätsmessung

Die Aktivitätsmessung erfolgte nach folgender Methode:

Zu 1,5 ml Chitinlösung (Sigma-Chitin Nr. C 3641; 4 mg Chitin/ml Aqua bidest.) wurden 0,38 ml Kulturüberstand gegeben. Der gesamte Ansatz wurde 1 h bei 45°C geschüttelt, so daß das Chitin fein suspendiert blieb. Dann wurde zentrifugiert und 0,5 ml aus dem Überstand wurden mit 1 ml Dinitrosalicylsäurereagenz gemischt. Das Reagenz besteht aus 10 g 3,5-Dinitrosalicylsäure, 300 g K-Na-Tartrat, 16 g NaOH in 1 l Wasser. Die Lösung ist lichtempfindlich.

Die Mischung wurde 5 min. in siedendem Wasser erhitzt, abgekühlt, 1:10 mit Wasser verdünnt und im Photometer bei 530 nm gegen einen Leerwert gemessen. Der Leerwert bestand aus 0,38 ml hitzeinaktiviertem Kulturüberstand (5 min. in siedendem Wasser), der mit 1,5 ml Chitinlösung gemischt und dann wie eine normale Probe behandelt wurde. Als 1 Unit (U) wurde die Enzymaktivität definiert, die in dem oben beschriebenen Test 1 $\mu$mol pro Minute an reduzierenden Zuckerresten aus Chitin freisetzt.

**Beispiel 3**

Kultivierung von Streptomyces spec. DSM 4666 im Fermenter

Streptomyces spec. DSM 4666 wurde wie in Beispiel 1 kultiviert. Ein 10 l Fermenter wurde mit 1 % Vorkultur beimpft. Die Fermenter-Nährlösung bestand aus:

| | |
|---|---|
| Krabbenchitin (Sigma C 3387) | 5 g/l |
| trockenes Fusarium-Myzel (DSM 2018) | 2 g/l siehe |
| " Penicillium-Myzel (DSM 1079) | 2 g/l Beispiel 4 |
| Hefeextrakt | 0,1 " |
| Cornsteep | 7 " |
| $Na_2HPO_4 \cdot 2H_2O$ | 1,8 " |
| $(NH_4)_2SO_4$ | 1 " |
| $K H_2PO_4$ | 0,5 " |
| $K_2HPO_4$ | 1 " |
| $MgSO_4 \cdot 7 H_2O$ | 0,5 " |
| $FeSO_4 \cdot 7 H_2O$ | 0,2 " |
| pH 7,7 | |

Es wurde bei einer Temperatur von 30°C inkubiert und mit 0,5 VVM belüftet. Nach 3 Tagen konnte eine Lyseaktivität von 206 U/l im Kulturfiltrat gemessen werden. Die Zellen wurden abgeschleudert, der Überstand gefriergetrocknet und als Enzympräparat (EP) verwendet.

**Beispiel 4**

Isolierung von Pilz-Zellwänden

Pilze der folgenden Spezies wurden in flüssigen bekannten Pilznährlösungen kultiviert:

fusarium oxysporium      DSM 2018
Penicillium roqueforti      DSM 1079
Botrytis cinerea      DSM 877
Geotrihum candidum      CBS 18767
Byssochlamus nivea      CBS 60871
Penicillium patulum      DSM 62862

Die Myzelien wurden geerntet, gewaschen, zerkleinert und 24 h mit der Protease Alcalase von Novo Industri behandelt. Anschließend wurde das Myzel erneut gewaschen und getrocknet.

**Beispiel 5**

Lysewirkung auf Pilzzellwände

Zellwände von Pilzen wurden wie in Beispiel 1 beschrieben isoliert. Zur Herstellung des Zellwandagars werden 0,4 g/l Pilzzellwände und 20 g/l Agar in 1 l 50 mmol/l Citronensäure-phosphatpuffer (pH 5,0) suspendiert bzw. gelöst. Die Bestandteile werden gemischt und autoklaviert. Vor dem Ausgießen in Petrischalen werden 0,6 g/l $NaN_3$ steril zugesetzt. In den Agar werden 8 mm runde Löcher gestanzt und in diese zum Vergleich je 100 μl folgender Enzymlösungen pipettiert:
- Enzympräparat nach Beispiel 3 1,43 mU/mg Protein•ml
- Chitinase Str.griseus (Sigma Nr. C 6137) 1,9 mU/mg Protein•ml
- Protoplast-forming-enzyme von Helix pomatia, Boehringer Mannheim Nr. 1010085      0,5 mU/mg Protein•ml

Die Enzymeinheiten beziehen sich auf den verwendeten Lysetest aus Beispiel 2.

Die Platten wurden 2 h bei 4°C aufbewahrt und anschließend 24 h bei 30°C inkubiert. Danach wurden folgende Lysehöfe (mm) abgelesen.

| Zellwandpolymer | Enzympräparat (Beispiel 3) | Chitinase | Protoplast-forming-enzyme |
|---|---|---|---|
| Chitin Sigma Nr. C 3641 | 16 | 14 | 14 |
| Fusarium oxysporium DSM 2018 | 20 | 15 | 19 |
| Penicillium roqueforti DSM 1079 | 18 | - | - |
| Botrytis cinerea DSM 877 | 19 | - | 18 |
| Geotrichum candidum CBS 18767 | 16 | - | - |
| Byssochlamus nivea CBS 60871 | 18 | - | - |
| Penicillium patulum DSM 62862 | 15 | - | 16 |
| - = keine Lysis | | | |

**Beispiel 6**

a) Temperaturresistenz

Der Enzymextrakt aus Beispiel 3 wurde bei 50, 60 und 70°C in Citronensäurephosphatpuffer 50 mmol.l (pH 5,0) inkubiert. Nach verschiedenen Zeiten wurden 50 µl Proben entnommen und in ausgestanzte Löcher (8 mm Durchmesser) einer Pilzzellwandagarplatte gefüllt. Die Platten hatten folgende Zusammensetzung:
3 g Pilzzellwände
18 g Agar
Die Platten wurden 24 h bei 30°C inkubiert. Dann wurden die Lysehofdurchmesser ausgemessen. Es ergaben sich folgende Werte:

| Penicillium roqueforti DSM 1079 (Lysehofdurchmesser in mm) | | | |
|---|---|---|---|
| Zeit [min] | Temperatur [0°C] | | |
| | 50 | 60 | 70 |
| 0 | 24 | 23 | 24 |
| 15 | 23 | 22 | 17 |
| 30 | 24 | 22 | 15 |
| 45 | 24 | 21 | 15 |
| 60 | 23 | 20 | KL |
| 75 | 23 | 16 | KL |
| 90 | 24 | 15 | KL |
| 105 | 23 | KL | KL |
| 120 | 21 | KL | KL |
| 135 | 22 | KL | KL |
| 150 | 20 | KL | KL |
| 165 | 20 | KL | KL |
| 180 | 18 | KL | KL |
| 195 | 18 | KL | KL |
| KL = keine Lyse | | | |

| Fusarium oxysporium DSM 2018 (Lysehofdurchmesser in mm) | | | |
|---|---|---|---|
| | Temperatur [°C] | | |
| Zeit [min.] | 50 | 60 | 70 |
| 0 | 24 | 24 | 24 |
| 15 | 24 | 24 | 23 |
| 30 | 25 | 25 | 22 |
| 45 | 24 | 24 | 22 |
| 60 | 24 | 23 | 20 |
| 75 | 25 | 24 | 23 |
| 90 | 24 | 20 | 17 |
| 105 | 24 | 15 | 15 |
| 120 | 23 | 16 | KL |
| 135 | 22 | 17 | KL |
| 150 | 23 | 16 | KL |
| 165 | 22 | 14 | KL |
| 180 | 21 | 15 | KL |
| 195 | 20 | KL | KL |
| KL = keine Lyse | | | |

b) pH-Optimum

Der Lyseaktivitätstest wurde, wie in Beispiel 5 beschrieben, bei verschiedenen pH-Werten und Puffern durchgeführt. Die nachfolgende Tabelle zeigt die gemessenen Extinktionen bei 530 nm. Die Pufferkonzentration betrug jeweils 50 mmol/l.

| Puffer | pH | Extinktion |
|---|---|---|
| Natrium-Acetat | 4,0 | 0,788 |
| Natrium-Acetat | 5,0 | 0,799 |
| Citronensäure-Phosphat | 5,0 | 0,801 |
| Natriumacetat | 6,0 | 0,786 |
| Phosphat | 7,0 | 0,758 |
| Phosphat | 8,0 | 0,750 |
| Tris/HCl | 8,0 | 0,753 |
| Tris/HCl | 9,0 | 0,739 |
| Tris/HCl | 10,0 | 0,740 |
| Tris = Tris-(hydroxymethyl)-aminomethan | | |

c) pH-Stabilität

Der gefriergetrocknete Extrakt wurde in verschiedenen Puffern gelöst und unterschiedlich lange Zeit bei 30°C inkubiert. Dann wurde eine Probe entnommen und die Lyseaktivität wurde, wie in Beispiel 5, in Citronensäurephosphatpuffer pH 5,0 bestimmt. Es wurden folgende Werte gemessen:

| | | Inkubationszeit (h) | | | |
|---|---|---|---|---|---|
| Puffer | pH | 0 | 12 | 24 | 48 |
| Natrium-Acetat | 4,0 | 0,8 | 0,788 | 0,765 | 0,758 |
| Natrium-Acetat | 5,0 | 0,8 | 0,795 | 0,781 | 0,764 |
| Citronensäure-Phosphat | 5,0 | 0,8 | 0,788 | 0,779 | 0,758 |
| Natrium-Acetat | 6,0 | 0,8 | 0,782 | 0,765 | 0,756 |
| Phosphat | 7,0 | 0,8 | 0,755 | 0,738 | 0,716 |
| Phosphat | 8,0 | 0,8 | 0,748 | 0,708 | 0,701 |
| Tris/HCl | 8,0 | 0,8 | 0,749 | 0,698 | 0,654 |
| Tris/HCl | 9,0 | 0,8 | 0,742 | 0,694 | 0,658 |
| Tris/HCl | 10,0 | 0,8 | 0,738 | 0,638 | 0,629 |

**Ansprüche**

1. Pilz-lysierendes Enzymprodukt, erhältlich durch Kultivierung von Streptomyces spec. DSM 4666, mit einer
- Temperaturstabilität bis zu 70° C,
- einem Temperaturoptimum von 40 bis 60° C,
-einer pH-Stabilität im Bereich von pH 4,0 bis 10,0 und
- einem pH-Optimum im Bereich 4,5 bis 6.

2. Verfahren zur Herstellung des Pilz-lysierenden Enzymprodukts nach Anspruch 1, dadurch gekennzeichnet, daß Streptomyces spec. DSM 4666 auf einem Chitinhaltigen und/oder Oligomere von N-Acetyl-1,4-glucosamin enthaltenden Substrat kultiviert wird.

3. Verfahren nach Anspruch 2, dadurch gekennzeichnet, daß Streptomyces spec. DSM 4666 auf einem Substrat kultiviert wird, daß 0,01 bis 5 % Krabbenchitin enthält.

4. Verfahren nach Anspruch 2 oder 3, dadurch gekennzeichnet, daß bei 18 bis 40° C kultiviert wird.

5. Verfahren nach einem oder mehreren der Ansprüche 2 bis 4, dadurch gekennzeichnet, daß bei einem pH-Bereich von 5 bis 9 kultiviert wird.

6. Verwendung des Pilz-lysierenden Enzymprodukts nach Anspruch 1 zur Konservierung von mikrobiell verderblichen Produkten.